Europäisches Patentamt

European Patent Office

Office européen des brevets

(19)

(11) Veröffentlichungsnummer: **0 041 963**
**B1**

(12) **EUROPÄISCHE PATENTSCHRIFT**

(45) Veröffentlichungstag der Patentschrift:
**24.07.85**

(21) Anmeldenummer: **80902279.1**

(22) Anmeldetag: **08.12.80**

(86) Internationale Anmeldenummer:
**PCT/DE 80/00182**

(87) Internationale Veröffentlichungsnummer:
**WO 81/01648 (25.06.81** Gazette 81/15)

(51) Int. Cl.⁴: **A 61 B 10/00,** A 61 B 17/42,
A 61 B 17/36

(54) **VORRICHTUNG ZUR MESSERKONISATION DES GEBÄRMUTTERHALSES.**

(30) Priorität: **07.12.79 DE 2949278**

(43) Veröffentlichungstag der Anmeldung:
**23.12.81 Patentblatt 81/51**

(45) Bekanntmachung des Hinweises auf die Patenterteilung:
**24.07.85 Patentblatt 85/30**

(84) Benannte Vertragsstaaten:
**CH DE FR GB LI SE**

(56) Entgegenhaltungen:
**DE - A - 1 516 414**
**FR - A - 818 286**
**US - A - 3 345 981**
**US - A - 3 357 422**
**US - A - 3 452 741**
**US - A - 3 613 662**

(73) Patentinhaber: **SCHWARZE, Johannes,
Georgenstrasse 42, D-8000 München 40 (DE)**

(72) Erfinder: **SCHWARZE, Johannes, Georgenstrasse 42,
D-8000 München 40 (DE)**
Erfinder: **KORTLÄNDER, Rainer, Eisenfelden 66,
D-8261 Neuötting II (DE)**

(74) Vertreter: **Kühnemann, Klaus et al,
Sonderburgstrasse 36, D-4000 Düsseldorf 11 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

### Technisches Gebiet

Die Erfindung betrifft eine Vorrichtung zur Messerkonisation des Gebärmutterhalses mit einem bandförmigen flexiblen Messer, welches mit seinem in den Gebärmutterkanal einzuführenden ersten Ende am vorderen Ende einer Stange befestigt ist und an einem radial von der Achse der Stange abstehenden, auf der Stange geführten und relativ zu ihr axial hin- und herbewegbaren Schieber radial ausschwenkbar gehaltert ist.

Bei der Messerkonisation des Gebärmutterhalses handelt es sich um eine Methode zur Entnahme einer Gewebeprobe am Gebärmutterhals zwecks Feststellung möglicher krankhafter Veränderungen und gleichzeitig zur therapeutischen Beseitigung von krankhaftem Gewebe am Gebärmutterhals.

### Stand der Technik

Bei einer bekannten Vorrichtung der vorstehend angegebenen Gattung (US-PS 3 613 662) ist an einem drehbeweglichen Stab zum Einführen in den Gebärmutterhals ein radial abstehender Ausleger angebracht, und das Messer erstreckt sich vom Vorderende des Stabes über den Ausleger bis zu zwei Halterungen, die nahe dem Hinterende des Stabes an einer pistolenartigen Handhabe befestigt sind. Hierbei muss das Messer vor dem operativen Eingriff in eine bestimmte fixe Stellung gebracht werden, welche den Radius des auszuschneidenden Konus festlegt. Es stehen drei Schlitze für drei verschiedene Radien zur Verfügung. Die Enden des Messers bleiben dabei stets fest eingespannt, d.h. die Grundstellung des Messers ist unveränderlich, und nur der Verlauf seines mittleren Abschnittes kann mehr oder weniger gekrümmt werden. Hiermit ist der Nachteil einer bauchigen Biopsie verbunden, was dazu führt, dass bei der Konisation auch Gewebe aus dem Muskel entnommen wird, aus dem eigentlich keine Gewebeproben entnommen werden sollen. Überdies besteht die Gefahr, dass mit der bekannten Vorrichtung auch die grösseren Blutgefässe im seitlichen Gebärmutterhalsbereich geöffnet werden, womit entsprechende Blutungen verbunden sind.

Bei einer weiteren bekannten Vorrichtung (US-PS 3 345 981), welche als Handgerät ohne eigenen Antrieb ausgebildet ist, sitzt das Vorderende des Messers am Vorderende einer Stichelstange, von wo aus das Messer in einem nicht verstellbaren Winkel zu einem radial abstehenden Messerhalter verläuft, an dem es mittels einer Klammer befestigt ist. Hiermit ist der Nachteil verbunden, dass der Operateur beide Hände benötigt, um die erforderlichen Stich- und Schneidbewegungen auszuführen.

Auch bei einem anderen bekannten Instrument zur Konisation der Portio ist das Messer in einem bestimmten Konuswinkel starr gehalten, so dass eine Anpassung der zu entnehmenden Gewebeprobe bezüglich ihrer Form und Grösse an die individuelle Ausdehnung der Gewebeveränderungen auf der Oberfläche des Muttermundes nicht möglich ist.

Während die bisher beschriebenen bekannten Vorrichtungen zur Durchführung der sogenannten kalten Konisation ausgeführt sind, gehören auch Geräte zur sogenannten Elektrokonisation zum Stand der Technik, bei denen der zu untersuchende Konus am Gebärmutterhals anstelle eines schneidenden Messers mit einem Hochfrequenz-Glühdraht entnommen wird (Hochfrequenzkaustik). Diese Methode ist aber wegen der damit verbundenen Thermo-Koagulation der Schnittränder histologisch unbefriedigend, weil diesen nicht mit Sicherheit angesehen werden kann, ob gesundes oder krankhaftes Gewebe angeschnitten wurde. In diesen Bereich gehört auch ein chirurgisches Instrument für die Konisierung des Gebärmutterhalses (DE-OS 2 309 205), mit dem ein Konus aus der Portio gelöst wird, dessen Schnittränder hitzedenaturiert, also verkocht oder verkohlt sind. Eine Manövrierbarkeit in bezug auf die seitlichen Exzisionsränder während des Konisierens ist nicht gegeben.

### Darstellung der Erfindung

Angesichts des beschriebenen Standes der Technik liegt der Erfindung die Aufgabe zugrunde, eine vollmechanisierte und weitgehend automatisch arbeitende Vorrichtung zu schaffen, mit der im Gegensatz zu den bekannten Geräten histologisch aussagekräftige und damit therapeutisch befriedigende Gewebeproben von individuell bestimmter Konusform und -grösse mit regelmässiger Sicherheit zu gewinnen sind. Dabei soll die Messerkonisation als solche durch wesentlich erleichterte Handhabung auch häufiger zur wirklich vollständigen Entfernung der Gewebeveränderungen angewendet werden können.

Die erfindungsgemässe Lösung dieser Aufgabe einschliesslich vorteilhafter Ausgestaltungen und Weiterbildungen ergibt sich aus den Patentansprüchen, welche dieser Beschreibung nachgestellt sind.

Die erfindungsgemässen Vorschläge bringen zahlreiche Vorteile mit sich. Insbesondere kann bei dem Eingriff immer vom Gebärmutterhalskanal ausgegangen werden, indem von dort aus zunächst eine radiale Einschnittbewegung durchgeführt wird, die an ihrem äusseren Ende in eine kegelförmige Kreisbewegung übergeht. Dies ermöglicht in allen Fällen vergleichsweise symmetrische konische Proben, womit unter anderem verstärkten Blutungen durch Anschneiden entfernterer Blutgefässe vorgebeugt wird. Aufgrund der Erfindung geschieht die ganze Konisierung auch besonders schonend, weil Zerr- und Reissbewegungen ausgeschlossen sind. Durch die nach einer Ausführungsform der Erfindung vorgesehene Hochfrequenzkaustik ausschliesslich an der Aussenseite des Trennmessers kann die Schnittfläche des im Körper der Patientin verbleibenden Gewebeteils blutungsstillend versiegelt und trotzdem eine Probe zur Untersuchung gewonnen werden, deren Schnittfläche ihrerseits nicht koaguliert und somit histologisch einwandfrei auswertbar ist.

Kurze Beschreibung der Zeichnungen

In der Zeichnung sind zwei Ausführungsbeispiele der Erfindung wiedergegeben, welche
nachstehend beschrieben werden. Es zeigen:

Fig. 1 eine schaubildlich-schematische seitliche
Innenansicht der Vorrichtung,

Fig. 1a eine seitliche Ansicht des das Messer
tragenden vorderen Teils der Vorrichtung,

Fig. 2 einen Schnitt nach Linie II–II in Fig. 1a,

Fig. 3 einen axialen Längsschnitt durch das
Messerende der Vorrichtung,

Fig. 4 einen vergrösserten Schnitt nach Linie
IV–IV in Fig. 2,

Fig. 5 den Gebärmutterhals mit dem ausgeschnittenen Konus in schaubildlich-schematischer Darstellung,

Fig. 6–9 den Ablauf der Konisation anhand von
vier verschiedenen Messerstellungen am Gebärmuttermund,

Fig. 10 eine schaubildlich-schematische Seitenansicht eines zweiten Ausführungsbeispiels
der Vorrichtung, und zwar entsprechend Fig. 1,
linker Teil, und Fig. 1a,

Fig. 11 einen Schnitt nach Linie XI–XI in Fig. 10,

Fig. 12 eine Seitenansicht der vorderen Messerlagerung bei Ausführung für Elektrokaustik,

Fig. 13 eine Draufsicht auf den Gegenstand von
Fig. 12,

Fig. 14 einen Querschnitt nach Linie XIV–XIV in
Fig. 13.

Zwei Wege zur Ausführung der Erfindung

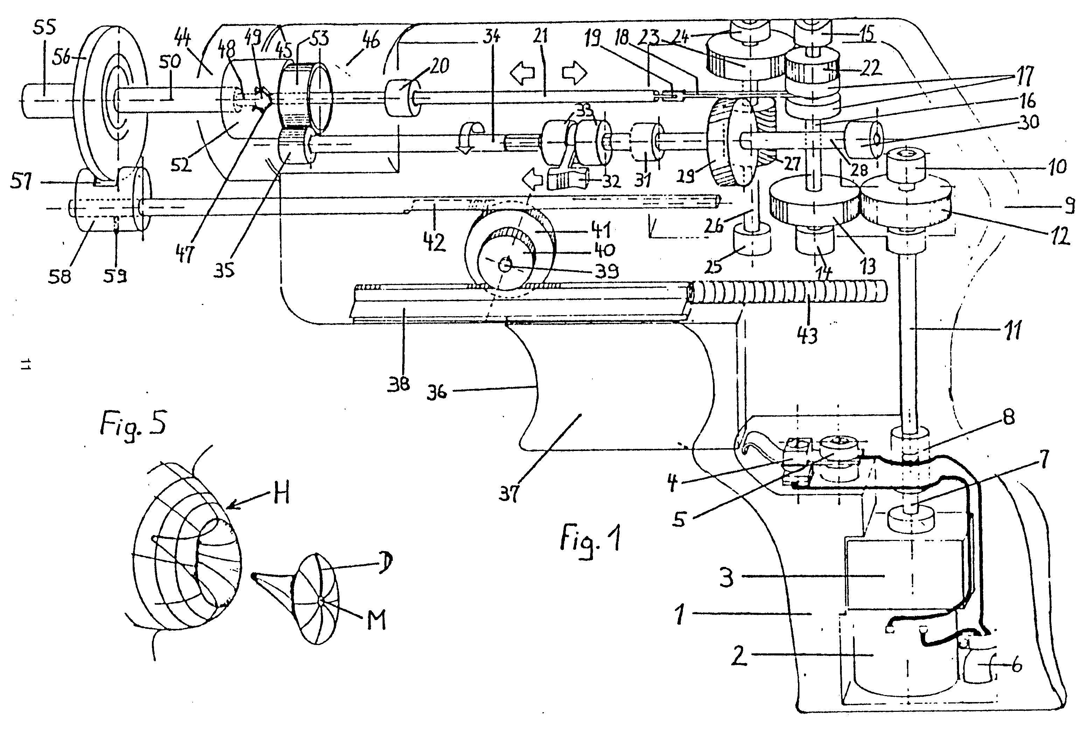

Die Figuren 1 und 1a sind nur im Zusammenhang miteinander verständlich, da die Wellenenden des in Figur 1a gezeigten Vorderteils des
Gerätes mit den aus dem in Figur 1 abgebildeten Gehäuse hervorstehenden Wellenstümpfen
(oben) einstückig verbunden sind. Die Vorrichtung
als solche hat die äussere Form einer Pistole. Im
Pistolengriff 1 ist ein elektrischer, pneumatischer
oder sonstiger geeigneter Antriebsmotor 2 mit
einem Untersetzungsgetriebe 3 untergebracht.
Der hier mit 7000 Umdrehungen pro Minute umlaufende Hochfrequenzmotor 2 ist über einen
Schalter 4, 5 und ein Stromzuleitungskabel 6 an
eine Netzsteckdose anschliessbar. Das Getriebe
3 untersetzt die Motordrehzahl im Verhältnis
60 : 1. Die daher mit 116,6 Umdrehungen pro
Minute umlaufende Getriebewelle 7 ist mittels
Kupplung 8 mit einer in das Pistolengehäuse 9 bis
zu einem Lager 10 hineinführenden Antriebswelle
11 verbunden. Auf dieser sitzt ein Zahnrad 12, das
mit einem gleichgrossen Zahnrad 13, also im Verhältnis 1 : 1, kämmt, welches auf einer in den Endlagern 14 und 15 umlaufenden Kurbelwelle 16
sitzt. An der Kurbel 17 greift eine Pleuelstange 18
an, die mittels Gabelgelenks 19 mit einer zum
Gerätkopf hinführenden und dort durch ein Lager
20 hindurchgeführten Stichelstange 21 verbunden
ist.

Auf der Kurbelwelle 16 sitzt zwischen Kurbel 17
und Endlager 15 ein Zahnrad 22, das kleiner ist als das auf der gleichen Kurbelwelle 16 sitzende
Zahnrad 13 und mit einem Zahnrad 23 gleichen
Durchmessers wie das Zahnrad 13, also untersetzend, auf einer parallel zur Kurbelwelle 16 in den
Endlagern 24 und 25 verlaufenden Welle 26
kämmt. Auf der Welle 26 sitzt eine Schnecke 27,
die in ein auf der quer dazu zum Gerätkopf hinführenden Welle 28 sitzendes Schneckenrad 29 eingreift. Die Welle 28 läuft hinten in einem Endlager
30 und ragt vorn durch ein Lager 31 hindurch, um
mittels einer von Hand durch Betätigen eines seitlich aus dem Pistolengehäuse 9 herausragenden
Hebels 32 ein- und ausrückbaren Kupplung 33 an
eine zum Gerätkopf hinführende Welle 34 anzuschliessen. Auf dem Vorderende dieser Welle 34
sitzt ein Zahnrad 35.

An der Unterseite des Pistolengehäuses 9 befindet sich – gewissermassen als Pistolenabzugshahn – ein vom Zeigefinger der den Pistolengriff
1 haltenden Hand des Operateurs zu umfassender, mit konkaver Angriffsrundung 36 versehener
Riegel 37, der mit einer oberen waagerechten, in
Längsrichtung des Pistolengehäuses 9 verschiebbaren Zahnstange 38 in dieses eingreift und dort
mit einem quer zum Pistolengehäuse 9 auf einer
Welle 39 gelagerten Zahnrad 40 kämmt. Auf der
gleichen Welle 39 sitzt ein Zahnrad 41 grösseren
Durchmessers als das Zahnrad 40. Das Zahnrad
41 greift – also mit Untersetzung – in eine darüber
parallel zur Zahnstange 38 verlaufende, an ihrer
Unterseite ebenfalls zahnstangenartig ausgebildete Stange 42 ein, die vorn aus dem Gerätkopf
herausragt. Gegen das Hinterende der Zahnstange 38 stützt sich eine andererseits im Pistolengehäuse 9 gelagerte Rückholschraubenfeder 43 ab.
Der wie ein Abzugshahn zu betätigende Riegel 37
hat gegen die Feder 43 einen Hub von 30 mm.
Dabei schiebt er sich mit der Unterseite seines
Hinterendes über den federnd niederdrückbaren
Stromschalterpol 4, der sich dabei auf den festen
Gegenpol 5 setzt und den Stromkreis zwischen
dem Zuleitungskabel 6 und dem Elektromotor 2
schliesst.

Die in die Scheide einzuführenden Teile der
Vorrichtung sind zwecks Reinigung und Desinfizierung vom Gerätkopf abnehmbar und auf ihn
mittels einer Überwurfkappe 44 aufsteckbar. In
dem die Überwurfkappe 44 aufnehmenden Gerätkopf 45 befindet sich eine Kammer 46, in die die
durch das Lager 20 hindurchlaufende Stichelstange 21 hineinragt. Sie endet darin mit einer federnden Steckhülse 47, in die das rückwärtige Ende
der in die Scheide einzuführenden Stichelstange
48 mit einem Kugelkopf 49 lösbar eingreift. Die
Stichelstange 48 ist von einem gleichachsigen
Rohr 50 umgeben, das durch ein Lager in der Mitte
der Überwurfkappe 44 mit einer aufgekeilten zylindrischen Büchse 52 in die Kammer 46 hineinragt. Am Aussenumfang ihres hinteren Teils trägt
die Büchse 52 eine Ringverzahnung 53, mit der
das im Durchmesser kleinere, auf dem Vorderende der Welle 34 sitzende Zahnrad 35 kämmt. Dabei läuft das Rohr 50 mit 3 Umdrehungen pro
Minute im Uhrzeigersinn um.

Das Rohr 50 ist von einem achsparallelen Rohr 55 umgeben, an dessen Hinterende ein Flansch 56 sitzt, welcher drehbar in die Nut 57 einer am Vorderende der Stange 42 sitzenden Klaue 58 eingreift. Zum Lösen des in die Scheide einzuführenden Teils des Gerätes ist die Klaue 58 mit einem radial in die Stange 42 eingreifenden und zurückziehbaren Stift 59 fixiert, also von der Stange 42 abstreifbar, so dass der Flansch 56 des Rohres 55 dann freigegeben wird.

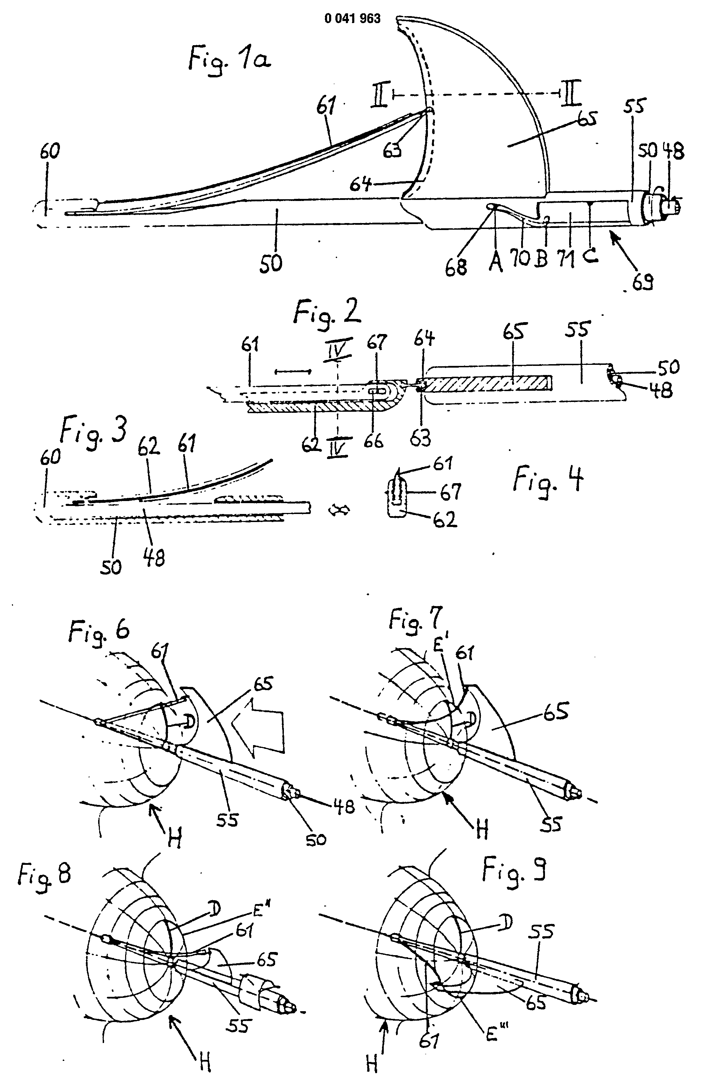

Das in den Gebärmuttermund M des Gebärmutterhalses H (Fig. 5) einzuführende Vorderende des Rohres 50 übergreift die Stichelstange 48 mit einer Kappe 60, die gleichzeitig das Vorderende eines bandförmigen Messers 61 trägt (Fig. 3). Das 70 mm lange Messer 61 ist flexibel und bis auf seine Schneidkante mit einem Kunststoffmantel 62 umhüllt (Fig. 4), der zur Führung des Messers bei seiner kurzhubigen und schnellen Schneidbewegung bestimmt ist. Am Hinterende des Messers sitzt ein Kugelkopf 63, der in einer entsprechenden schwalbenschwanzförmigen Nut 64 an der Stirnseite eines sichelförmigen Schiebers 65 gleitend geführt ist (Fig. 2). Der Schieber 65 sitzt radial abstehend aussen auf dem vorderen Bereich des Rohres 55. Zum Längenausgleich beim Durchbiegen des Messers 61 ist dessen Hinterende mittels einer axialen Längsnut 66 an einem quer durch den Kunststoffmantel 62 hindurchgesteckten Stift 67 geführt. Am Umfang des inneren Rohres 50 steht radial ein Mitnehmerstift 68 ab, der in eine Steuerkulisse 69 in dem umhüllenden äusseren Rohr 55 eingreift. Die Kulisse 69 besteht aus einem schräg zur Achse des Rohres 55 im Uhrzeigersinn verlaufenden Führungskanal 70 mit nach hinten anschliessendem rechteckigen Ausschnitt 71 (Fig. 1a). Ihre Wirkungsweise wird in der nun folgenden Schilderung der Betätigungs- und Arbeitsweise der Vorrichtung erklärt.

Wenn die Vorrichtung mit der Kappe 60 voran in den Gebärmutterhalskanal eingeführt wird, liegt das Messer 61 in seiner ganzen Länge nahezu parallel an dem den Schieber 65 tragenden äusseren Rohr 55 an. Der Mitnehmerstift 68 befindet sich an der Stelle A der Steuerkulisse 69. Die Kupplung 33 ist durch Betätigen des Hebels 32 ausgerückt, so dass die Welle 34 und somit auch das innere Rohr 50 stillstehen. Nach Einschalten des Motors 2 durch Antippen an der Rundung 36 des Riegels 37 und damit Schliessen des Schalters 4, 5 führt die Stange 21 und damit ihre Fortsetzung, die Stange 48, an der vorn das Vorderende des Messers 61 sitzt, dank dem sich drehenden Kurbeltrieb 17 eine kurzhubige hin- und hergehende, also Stichelbewegung, aus. Gleichzeitig gleitet durch das Antippen des Riegels 37 und die dadurch bewirkte Vorwärtsbewegung der Stange 42 das äussere Rohr 55 mit dem Schieber 65 vorwärts (Fig. 1), wodurch das Messer 61 mit seinem hinteren Kugelkopf 63 in der Schiebernut 64 radial ausschwenkt (Fig. 1a). Dabei hat sich das vorwärts verschobene äussere Rohr 55 infolge Gleitens des Mitnehmerstiftes 68 im Führungskanal 70 der Steuerkulisse 69 bis zur Stellung B im rechteckigen Ausschnitt 71 um eine Viertelumdrehung im Uhrzeigersinn gedreht. Ein gerader radialer Einschnitt D (Fig. 5 und 6) des Messers 61 in den Gebärmutterhals H ist vollzogen.

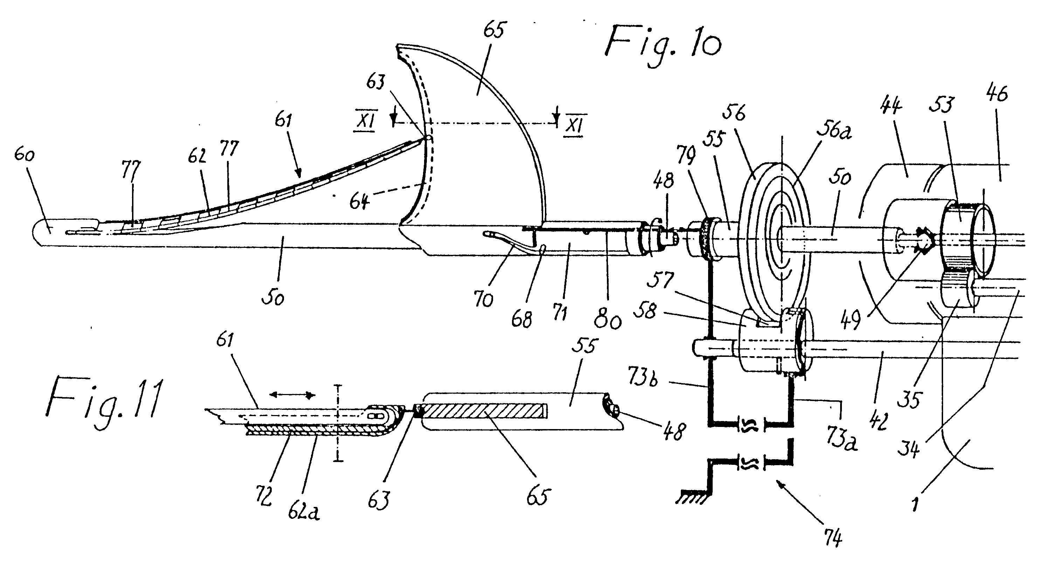

Nun wird die Kupplung 33 eingerückt, und das innere Rohr 50 beginnt, sich im Uhrzeigersinn zu drehen. Dabei durchläuft das Vorderende des Messers 61 eine Vierteldrehung. Erst danach erfasst der Mitnehmerstift 68 in der Stellung C in der rechteckigen Aussparung 71 der Steuerkulisse 69 das äussere Rohr 55. Das lange flexible Bandmesser 61 hat sich dabei in seinem ganzen hinteren, für den Schnitt in Frage kommenden Bereich um seine Längsachse so verwunden, dass es nach vorhergehender Radialbewegung in die Kreisbewegung eingeschwenkt ist und unter synchroner Drehbewegung beider Rohre 50 und 55 die kreisförmige Ausschneidung E des Gewebekonus vornimmt. Das Messer 61 läuft also gewissermassen hinter seiner Führung am beweglichen Kugelkopf-Hinterende 63 in der Nut 64 des Schiebers 65 her (Fig. 7–9). In Figur 7 hat das Messer 61 seinen Kreisweg E' gerade kurz begonnen. In Figur 8 hat es etwa ein Viertel E'' davon zurückgelegt, und in Figur 9 hat es etwa drei Viertel E''' seines Kreisweges hinter sich, bis dann schliesslich der Gewebekonus herausgeschnitten ist (Fig. 5).

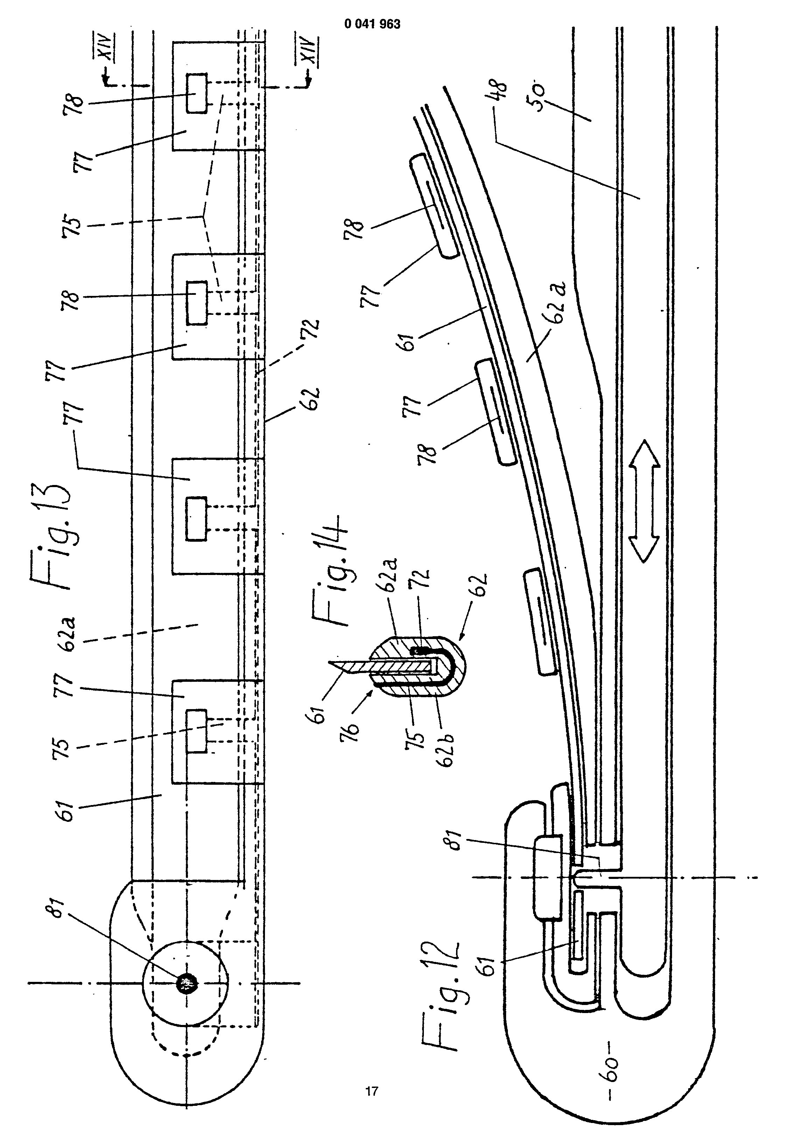

Die Figuren 10–14 stellen ein zweites Ausführungsbeispiel der Messerkonisationsvorrichtung dar, und zwar ist diese Vorrichtung zur Elektrokaustik eingerichtet, indem elektrischer Strom in die Messerscheide 62 eingeleitet wird, wozu in diese eine Stromleitschiene 72 eingebettet ist, welche über eine elektrische Leitung 73 mit einer Spannungsquelle 74 in Verbindung steht (Fig. 10). Von dieser Spannungsquelle aus wird der elektrische Strom über einen Leitungsabschnitt 73a zu der Klaue 58 geführt, in welche der Flansch 56 drehbar eingreift. In der Nut 57 der Klaue 58 wird ein Schleifkontakt zwischen dem Leitungsabschnitt 73a und dem Flansch 56 hergestellt; die entsprechende Schleifspur 56a ist aus Figur 10 ersichtlich. Von dem Flansch 56 aus fliesst der Strom über das Rohr 55 zum Schieber 65 und dessen schwalbenschwanzförmige Nut 64, in der Kugelkopf 63 des Messers 61 gleitet. Dabei ist der Kugelkopf 63 leitend mit der Stromleitschiene 72 verbunden, welche die Form einer schmalen Federstahlschiene hat. Deren Einlagerung in die Messerscheide 62 ergibt sich insbesondere aus Figur 14, welche einen Querschnitt durch Scheide und Messer zeigt.

Die im ganzen U-förmige Messerscheide weist einen Schenkel 62a auf, in dem sich die Stromleitschiene 72 befindet. Dieser Schenkel 62a hat über die ganze Länge der Scheide 62 einen im wesentlichen unveränderten Querschnitt, so dass die Stromleitschiene 72 dort gänzlich verdeckt ist und nach aussen hin nicht hervortritt. Infolgedessen kann im Bereich des Scheidenschenkels 62a kein elektrischer Kontakt mit Körpergewebeteilen stattfinden, an denen der Schenkel 62a sich entlangbewegt.

Zu dem anderen Schenkel 62b der Scheide 62 hin ist die Stromleitschiene 72 mit vorzugsweise einstückig angeformten Ansatzstücken 75 versehen, die sich in regelmässigen Abständen an der Stromleitschiene 72 befinden (Fig. 13). Diese Ansatzstücke erstrecken sich rechtwinklig von der Stromleitschiene 72 und sind innerhalb des die Schenkel 62a, 62b verbindenden U-Bogens der Scheide 62 ebenfalls U-förmig geführt und durchlaufen den Schenkel 62b in eingebetteter Form bis zu dessen Vorderkante, wo sie als Kontakt 76 frei leitend austreten. Zur Verbesserung der Gleiteigenschaften der Scheide 62 im Gewebe kann der Scheidenschenkel 62b, welcher die Ansatzstücke 75 enthält, gemäss den Figuren 12 und 13 zinnenartig gestaltet sein, so dass sich Finger 77 ergeben, in denen die Ansatzstücke 75 untergebracht sind und aus deren Vorderkante die Ansatzstücke 75 mit einem T-förmig verbreiterten Kontaktkopf 78 im wesentlichen bündig abschliessend hervortreten. Dadurch kann die mit dem Scheidenschenkel 62b in Berührung kommende Körpergewebeschicht mit den Wirkungen des elektrischen Stroms beaufschlagt werden.

Die Rückführung des über die Kontaktköpfe 78 (Fig. 13) in den Körper der Patientin eingeleiteten Stromes kann in an sich herkömmlicher Weise monopolar über eine breitflächige Kathode erfolgen, die in der Zeichnung nicht dargestellt und an irgendeiner geeigneten Stelle am Körper der Patientin angelegt worden ist. Wahlweise ist es jedoch auch möglich, den elektrischen Strom bipolar zu führen, d.h. innerhalb der Konisationsvorrichtung zur Spannungsquelle 74 zurückzuleiten. Hierzu wird die Spannung an das Messer 61 als zweiter Pol angelegt.

Wie sich aus Figur 10 ergibt, erfolgt die Heranführung des zweiten Poles über einen Leitungsabschnitt 73b zu einem Ringglied 79, an welches sich eine offene Kontaktschiene 80 anschliesst, die an der Oberkante der Steuerkulisse 69 für den Mitnehmerstift 68 angeordnet ist. Unter der Voraussetzung, dass der Mitnehmerstift 68 während seiner vorgesehenen Bewegungen bei der Konisation die Kontaktschiene 80 berührt, erfolgt die weitere Fortleitung des Stromes über diesen Stift und das den Stift tragende Rohr 50 zur Stichelstange 48, welche nahe ihrer Spitze oben einen Mitnehmerstift 81 trägt, der in eine Öffnung des Messers 61 eingreift und dadurch dessen oszillierende Bewegungen verursacht. Auf diese Weise ist der zweite Pol der Spannungsquelle 74 an das Messer 61 angeschlossen und damit das bipolare System hergestellt. Es versteht sich, dass die an verschiedene Pole angeschlossenen Bauteile gegeneinander isoliert sein müssen.

Durch die Ausbildung der Kontaktschiene 80 und deren Anordnung in der Steuerkulisse 69 ergibt sich die Wirkung, dass eine Stromzuführung zu den Kontakten 76, 78 und zum Messer 61 nur dann geschieht, wenn das Messer am oberen Ende des Schiebers 65 angekommen und in seine Kreisbahn eingeschwenkt ist, d.h., während des radialen Einschnittes D (Fig. 5 und 6) findet keine Elektrokaustik statt, weil der Mitnehmerstift 68 die Kontaktschiene 80 nicht berührt, so dass auch keine leitende Verbindung hergestellt und der Stromkreis nicht geschlossen ist. Eine Koagulation des radialen Einschnittes findet nicht statt.

Durch die besondere Ausbildung der Messerscheide 62 mit ihren elektrischen Kontakten 76, 78 wird nach dem Einschwenken des Messers in die Kreisbahn nur das im Körper der Patientin verbleibende Gewebe kaustisch verschlossen, während die Oberfläche des Gewebes der konischen Probe unverändert bleibt, so dass einerseits das Bluten stark eingedämmt werden kann, andererseits aber keinerlei Beeinträchtigung der Möglichkeit zur Untersuchung und Begutachtung des entnommenen Gewebes in Kauf genommen zu werden braucht, wie es bei der herkömmlichen Elektrokaustik der Fall ist. Durch die beschriebene Ausbildung wird ein kaustisch versiegelter Wundkrater mit glatter, regelmässiger Oberfläche zurückgelassen, wozu auch die zinnenartige Gestaltung des Schenkels 62b der Messerscheide 62 beiträgt, da hierdurch eine ganz gleichmässige Biegung des gesamten Messerorgans möglich ist. Dabei übernimmt die Stromleitschiene 72 zusätzliche die Aufgabe eines stabilisierenden Elementes in der Umscheidung des Messers 61, was zur ruhigen Messerführung während der konischen Schneidbahn E beiträgt, so dass auch etwaige Schwingungseinflüsse seitens des Messerantriebes ausgeschaltet sind.

Dies gilt insbesondere für den Fall, dass der Antrieb des Messers 61 durch einen elektromagnetischen Schwinger (nicht dargestellt) erfolgt, der das Messer mit ungefähr 50 bis 200 Hertz bei einem Hub von etwa 0,1 mm oszillieren lässt. Als Widerlager für die Messerhalterung kann dabei ein Gummikissen verwendet werden (nicht dargestellt). Mit diesem Antrieb ist der Vorteil verbunden, dass das in Figur 1 dargestellte Getriebe teilweise entfallen kann, womit das Gerät gewichtsmässig leichter wird.

Die in den Patentansprüchen, in der Beschreibung und in der Zeichnung offenbarten Merkmale des Anmeldungsgegenstandes können sowohl einzeln als auch in beliebigen Kombinationen untereinander für die Verwirklichung der Erfindung in ihren verschiedenen Ausführungsformen wesentlich sein.

**Patentansprüche**

1. Vorrichtung zur Messerkonisation des Gebärmutterhalses mit einem bandförmigen flexiblen Messer (61), welches mit seinem in den Gebärmutterkanal einzuführenden ersten Ende am vorderen Ende einer Stange (48) befestigt ist und an einem radial von der Achse der Stange abstehenden, auf der Stange (48) geführten und relativ zu ihr axial hin- und herbewegbaren Schieber (65) radial ausschwenkbar gehaltert ist, gekennzeichnet durch folgende Merkmale:

a) dass der Schieber (65), an dem das zweite Ende des Messers (61) gehalten ist, derart an einem Rohr drehbar geführt ist, dass sich die gekrümmte Fläche des flexiblen bandförmigen Messerblattes um eine zur Stange (48) parallele Längsachse verwinden lässt,

b) dass das Rohr koaxial zur Stange (48) und letztere umhüllend angeordnet ist, wobei das Rohr zur Konisation in Rotation versetzt werden kann,

c) und dass die Stange (48) zur Ausführung einer stichelartigen Bewegung des Messers (61) relativ zum Rohr axial hin- und herbewegbar ist.

2. Vorrichtung nach Anspruch 1, dadurch gekennzeichnet, dass die axial hin- und herbewegbare Stange (48) teleskopartig von einem ersten ebenfalls am Vorderende des Messers (61) angreifenden und in Umlauf versetzbaren Rohr (50) umgeben ist, welches seinerseits teleskopartig von einem relativ zum ersten Rohr (50) axial beweglichen und von ihm mitnehmbaren zweiten Rohr (55) umhüllt ist, das den radial abstehenden Schieber (65) trägt.

3. Vorrichtung nach Anspruch 2, dadurch gekennzeichnet, dass sich an dem zweiten den Schieber (65) tragenden äusseren Rohr (55) eine aus einem schräg zur Achse dieses Rohres (55) im Uhrzeigersinn verlaufenden Führungskanal (70) mit nach hinten anschliessendem rechteckigem Ausschnitt (71) bestehende Steuerkulisse (69) befindet, in die ein vom ersten Messerantriebsrohr (50) radial nach aussen abstehender Mitnehmerstift (68) derart eingreift, dass er sich bei in Ruhestellung nahezu parallel am Messerantriebsrohr (50) anliegendem Messer (61) am äusseren Ende des Führungskanals (70) der Steuerkulisse (69) befindet (Stellung A) und nach Einschalten der eine stichelartige Bewegung ausführenden Stange (48) sowie axialem Verschieben des den Schieber (65) tragenden zweiten Rohres (55) zwecks radialen Ausschwenkens des Messerhinterendes nach Ausführung einer Viertelumdrehung des Messerantriebsrohres (50) an die nächstliegende achsparallele Kante des Kulissenfensters (71) anschlägt (Stellung B) sowie nach Einschalten des Messerantriebsrohres (50) zum Umlauf im Uhrzeigersinn nach Ausführung einer Viertelumdrehung an die gegenüberliegende Kante des Kulissenfensters (71) anschlägt (Stellung C) und dann das den Schieber (65) tragende zweite Rohr (55) bei ausgeschwenktem Messerhinterende mitnimmt.

4. Vorrichtung nach Anspruch 2 oder 3, dadurch gekennzeichnet, dass der Antrieb für die Stichelstange (48), für das Messerantriebsrohr (50) und für das den Schieber (65) tragende zweite Rohr (55) in an sich bekannter Weise in einem pistolenförmigen Gehäuse (9) untergebracht ist, wobei sich der Antriebsmotor (2) im Pistolengriff (1) befindet.

5. Vorrichtung nach einem der Ansprüche 2 bis 4, dadurch gekennzeichnet, dass die Motorwelle (7, 11) eine Kurbelwelle (16) zur Übertragung hin- und hergehender Bewegung auf die vorn aus dem Gehäuse (9) austretende Stichelstange (48) und über ein Untersetzungsgetriebe (22, 23) einen Schneckentrieb (27, 29) antreibt, der über eine ausschaltbare Wellenkupplung (33) und ein weiteres Untersetzungsgetriebe (35, 53) das die Stichelstange (48) umgebende Messerantriebsrohr (50) antreibt, während ein den Motor (2) betätigender Schalter (37) in Form eines Pistolenabzugshahns gleichzeitig über einen gegen eine Rückholfeder (43) wirkenden untersetzten Zahnstangentrieb (38–42) mittels Klaue (57, 58) und Flansch (56) die Axialbewegung des den Schieber (65) für das Messerhinterende tragenden zweiten Rohres (55) bewirkt.

6. Vorrichtung nach einem der Ansprüche 2 bis 5, dadurch gekennzeichnet, dass seine aus dem Antriebsgehäuse (9) herausragenden Teile abnehmbar sind, indem die aus einer federnden Steckhülse (47) lösbare Stichelstange (48) und das mit einer am Aussenumfang verzahnten Büchse (52, 53) auf das Ritzel (35) seiner Antriebswelle (34) aufsteckbare Messerantriebsrohr (50) durch eine abnehmbare Überwurfkappe (44) herausgeführt sind, während die den Flansch (56) des den Schieber (65) tragenden zweiten Rohres (55) erfassende Klaue (57, 58) am Vorderende der aus dem Gehäuse (9) herausragenden Stange (42) des Zahnstangentriebes (38–42) abnehmbar befestigt ist.

7. Vorrichtung nach Anspruch 1, dadurch gekennzeichnet, dass das Messer (61) an seinem Hinterende mittels eines Kugelkopfes (63) in einer Schwalbenschwanznut (64) an der Stirnseite des Schiebers (65) gleitend und verwindbar geführt ist.

8. Vorrichtung nach Anspruch 1, dadurch gekennzeichnet, dass das Messer (61) mit Ausnahme seiner Schneidkante von einer Schneide in Form eines flexiblen Kunststoffmantels (62) umhüllt ist.

9. Vorrichtung nach Anspruch 8, dadurch gekennzeichnet, dass das umhüllte Messer (61) zum Längenausgleich gegenüber dem Kunststoffmantel (62) bei seiner Durchbiegung an seinem Hinterende mittels einer Längsnut (66) an einem Querstift (67) des Kunststoffmantels (62) längsbeweglich gelagert ist.

10. Vorrichtung nach Anspruch 8, dadurch gekennzeichnet, dass sie in an sich bekannter Weise zur Elektrokaustik eingerichtet ist, indem der elektrische Strom in die Messerscheide (62) eingeleitet wird, wozu in diese eine Stromleitschiene (72) eingebettet ist, welche über eine elektrische Leitung (73) mit einer Spannungsquelle (74) in Verbindung steht.

11. Vorrichtung nach Anspruch 10, dadurch gekennzeichnet, dass die Stromleitschiene (72) mit leitenden Ansatzstücken (75) versehen ist, die sich zur Aussenseite der Messerscheide (62) hin erstrecken und ausschliesslich dort zum Kontakt austreten, so dass eine Thermo-Koagulation nur am Schnittrand des nicht entnommenen Gewebes erfolgt.

12. Vorrichtung nach Anspruch 10 oder 11, dadurch gekennzeichnet, dass die Stromzuführung zur Messerscheide (62) während der Bewegung des Messers (61) zum Vollzug des geraden radialen Einschnittes (D) unterbrochen ist.

13. Vorrichtung nach Anspruch 10 oder 11, dadurch gekennzeichnet, dass die Aussenseite der Messerscheide (62) zur Schneidkante des Messers (61) hin zinnenartig gestaltet und mit Fingern (77) versehen ist, in die sich die leitenden Ansatzstücke (75) der Stromleitschiene (72) hinein erstrecken und aus deren Spitzenbereich sie zum Kontakt mit dem Gewebe austreten.

14. Vorrichtung nach einem der Ansprüche 10 bis 13, dadurch gekennzeichnet, dass die Stromversorgung der Stromleitschiene (72) in der Messerscheide (62) über den mittels Gleitkontakten an die Spannungsquelle angeschlossenen Schieber (65) vorgesehen ist.

15. Vorrichtung nach einem der Ansprüche 3, 12 oder 14, dadurch gekennzeichnet, dass der eine Pol der Stromversorgung über eine in der Steuerkulisse (69) für den Mitnehmerstift (68) angeordnete offene Kontaktschiene (80) geführt ist, so dass der Strom nur dann fortgeleitet wird, wenn der Mitnehmerstift (68) während seiner Bewegung zur Berührung mit der Kontaktschiene (80) gekommen ist, nämlich im Augenblick des Einschwenkens des Messers (61) auf die konische Kreisbahn.

16. Vorrichtung nach Anspruch 11, dadurch gekennzeichnet, dass die Stromleitschiene (72) mit ihren leitenden Ansatzstücken (75) einstückig und im Querschnitt U-förmig ausgebildet ist und aus das flexible Messer (61) stabilisierendem Federstahl besteht, wobei die zum Kontakt mit dem Gewebe austretenden Enden der Ansatzstücke (74) insbesondere T-förmig verbreitert sind.

17. Vorrichtung nach einem der Ansprüche 10 bis 16, dadurch gekennzeichnet, dass die Stromversorgung der Stromleitschiene (72) im Gerät einpolig ausgebildet ist, indem der zweite Pol über eine Körperkathode geführt ist.

**Claims**

1. A device for blade conization of the mouth or cervix of the uterus, with a band-like flexible blade (61), the first end of which being introducable into the uterus cervical canal is seated on the front end of a rod (48), and which is supported in being swung radially outwardly on a slide (65) which projects radially from the axis of the rod (48) and is guided on the rod (48) and is likewise axially movable back and forth relative to the rod (48), characterized in

a) the slide (65), on which the second end of the blade (61) is supported, is pivotable guided on a tube in such a manner that the curved surface of the flexible band-like blade (61) is twistable about a longitudinal axis parallel to the rod (48),

b) the tube is arranged coaxial with the rod (48) and coating the latter, the tube being capable of being rotated for conization

c) and the rod (48) for executing the stitching-like movement of the blade (61) is axially movable back and forth relative to the tube.

2. A device according to claim 1, characterized in that the rod (48), which is axially movable back and forth, is surrounded telescopically by a first tube (50) which is variably rotatable and likewise engages the front end of the blade (61), said tube in turn being telescopically surrounded by a second tube (55) which is axially movable relative to the first tube (50), and is also capable of being taken along by the tube (50), with the tube (55) supporting the radially projecting slide (65).

3. A device according to claim 2, characterized in that on the second outer tube (55), which supports the slide (65), there is located a control guide (69) comprising a guide channel (70), which extends at an incline to the axis of this tube (55) in a clockwise direction, and a rearwardly adjoining rectangular cutout (71); into this control guide (69) engages a follower pin (68), which projects radially outwardly from the first blade drive tube (50), in such a way that, with the blade (61) in the rest position being located nearly parallel along the blade drive tube (50), the follower pin is located at the outer end of the guide channel (70) of the control guide (69) (position A); after engaging the rod (48) executing the stitching-like movement, as well as axially shifting the second tube (55), which supports the slide (65), for the purpose of radially swinging or pivoting out the rear end of the blade, the follower pin (68), after the blade drive tube (50) carries out a ¼ rotation, engages the nearest axially parallel edge of the guide window or cutout (71) (position B); after engaging the blade drive tube (50) for rotation in the clockwise direction, after carrying out a ¼ rotation, the follower pin (68) engages the oppositely located edge of the guide window (71) (position C); and then the follower pin (68) takes along the second tube (55), which supports the slide (65), with the rear end of the blade being swung or pivoted out.

4. A device according to claim 2 or 3, characterized in that the drive for the rod (48), for the blade drive tube (50), and for the second tube (55) which supports the slide (65) is accommodated in a known manner in a pistol-shaped housing (9), with the drive motor (2) being located in the pistol grip (1).

5. A device according to one of the claims 2 through 4, characterized in that the motor shaft (7, 11) drive a crankshaft (16) for transferring reciprocal movement to the rod (48) which projects out of the front of the housing (9), and also drives a worm drive (27, 28) by means of a reduction gearing (22, 23), which drives the blade drive tube (50), which surrounds the rod (48), by means of a disconnectable shaft coupling (33) and a further reduction gearing (35, 53), while a switch (37) in the form of a pistol-trigger mechanism actuates the motor (2) and simultaneously effects the axial movement of the second tube (55) which supports the slide (65) for the rear end of the blade, with such axial movement being effected by means of a cluth (57, 58) and flange (56) through the intervention of a geared down rack drive gearing (38–42) which acts against a return spring (43).

6. A device according to one of the claims 2 through 5, characterized in that those parts of the device which project from the drive housing (9) are removable; some of these parts are guided out of a removable cap (44), including the rod (48), which is detachable from a resilient plug sleeve (47), and the blade drive tube (50), which, with a sleeve (52, 53) toothed on the outer periphery, is insertable on the gear wheel (35) of its drive shaft (34); the clutch (57, 58), which engages the flange (56) of the second tube (55) which supports the slide (65), is removably fastened to the front end of the rod (42) of the rack drive gearing (38–42), which rod (42) projects from the housing (9).

7. A device according to claim 1, characterized in that the rear end of the blade (61) is guided slidably and in a twisted manner on the end face of the slide (65) in a dovetailed groove (64) by means of a spherical head (63).

8. A device according to claim 1, characterized in that the blade (61), with the exception of its cutting edge, is surrounded by a sheath in the form of a flexible synthetic material mantle (62).

9. A device according to claim 8, characterized in that, to adjust or equalize its length relative to the synthetic material mantle (62) during bending, the rear end of the surrounded blade (61) is longitudinally movably journalled on a transverse pin (68) of the synthetic material mantle (62) by means of a longitudinal groove (66).

10. A device according to claim 8, characterized in that it is equipped in a known manner for electro-cauterization, with the electrical current being introduced into the blade sheath (62), for which purpose a current-conducting strip (72) is embedded therein and is connected by means of an electrical line (73) with a voltage source (74).

11. A device according to claim 10, characterized in that the current-conducting strip (72) is provided with conductive extensions or projecting pieces (75) which extend to the outer side of the blade sheath (62), which is the only place they stick out for contact, so that a thermocoagulation only occurs along the cut edge of the tissue which was not removed.

12. A device according to claim 10 or 11, characterized in that the current supply to the blade sheath (62) is interrupted during the movement of the blade (61) for completion of the linear radial incision (D).

13. A device according to claim 10 or 11, characterized in that the outer side of the blade sheath (62) is scalloped toward the cutting edge of the blade (61) and has fingers (77) into which the conductive extensions (75) of the current-conducting strip (72) extend, and from the tip region of which they stick out for contact with the tissue.

14. A device according to one of the claims 10 through 13, characterized in that the current supply of the current-conducting strip (72) in the blade sheath (62) is supplied by means of the slide (65), which is connected to the voltage source by means of slide contacts.

15. A device according to one of the claims 3, 12 or 14, characterized in that one pole of the current supply is conducted by means of an exposed contact strip (80) arranged in the control guide (69) for the follower pin (68), so that the current is only conducted when the follower pin (68), during its movement, contacts the contact strip (80), namely at the moment of the pivoting or swinging-in of the blade (61) on the conical circular path.

16. A device according to claim 11, characterized in that the current-conducting strip (72), with its conductive extensions (75), is made integrally, has a U-shaped cross section, and comprises spring steel which stabilizes the flexible blade (61), whereby those ends of the extensions (75) which stick out for contact with the tissue are widened, especially so as to be T-shaped.

17. A device according to one of the claims 10 through 16, characterized in that the current supply of the current conducting strip (72) is embodied with a single pole in the device, with the second pole being conducted by means of a bodily cathode.

**Revendications**

1. Dispositif pour prélever un fragment de forme conique du tissu du col de l'utérus à l'aide d'un couteau (61) en forme de ruban flexible, qui est fixé, par sa première extrémité destinée à être introduite dans le canal de l'utérus, à l'extrémité avant d'une tige (48) et est maintenu, pivotant radialement, sur un coulisseau (65) qui s'étend radialement à partir de l'axe de la tige, qui est guidé sur la tige (48) et est déplaçable axialement par rapport à celle-ci, alternativement dans un sens ou dans l'autre, caractérisé:

a) en ce que le coulisseau (65), sur lequel la seconde extrémité du couteau (61) est maintenue, est guidé rotatif sur un tube, de telle façon que la face incurvée de la feuille de coupe en forme de ruban flexible s'enroule autour d'un axe longitudinal parallèle à la tige (48),

b) en ce que le tube est disposé coaxialement à la tige (48) de façon à entourer celle-ci, le tube étant apte à être déplacé en rotation pour effectuer le prélèvement d'un fragment conique,

c) et en ce que la tige (48) est déplaçable axialement dans un sens et dans l'autre par rapport au tube, afin de réaliser un mouvement de pénétration du couteau (61).

2. Dispositif selon la revendication 1, caractérisé en ce que la tige (48) déplaçable dans un sens et dans l'autre est entourée, à la façon d'un télescope, par un premier tube (50) déplaçable en rotation et qui est également en prise avec l'extrémité avant du couteau (61), et en ce que ce tube (50) est lui-même entouré, à la façon d'un télescope, par un second tube (55) qui est déplaçable axialement par rapport au premier tube (50) et est apte à être entraîné par celui-ci et qui porte le coulisseau (65) s'étendant radialement.

3. Dispositif selon la revendication 1, caractérisé en ce que, sur le second tube extérieur (55) portant le coulisseau (65), se trouve une coulisse de commande (69) ayant la forme d'un canal de guidage (70) s'étendant en biais, dans le sens des aiguilles d'une montre, par rapport à l'axe de ce tube (55) et avec une découpe rectangulaire (71) se raccordant par derrière, en ce qu'une broche d'entraînement (68) s'étendant radialement vers l'extérieur à partir du premier tube d'entraînement (50) du couteau est engagée dans cette coulisse (69), de telle façon qu'elle se trouve à l'extrémité extérieure du canal de guidage (70) de la coulisse de commande (69) (position A), lorsque le couteau (61) se trouve à peu près parallèle au tube d'entraînement (50) du tube en position de repos, qu'après mise en marche de la tige (48) réalisant un mouvement de pénétration, ainsi qu'après un déplacement axial du second tube (55) portant le coulisseau (65), en vue de réaliser un pivotement radial de l'extrémité arrière du couteau après rotation d'un quart de tour du tube d'entraînement (50), elle vienne buter contre le bord parallèle à l'axe situé le plus près de la fenêtre (71) de la coulisse (position B), qu'après mise en route du tube d'entraînement du couteau (50) en vue de sa rotation dans le sens horaire, elle vienne buter, après rotation d'un quart de tour, contre le bord opposé de la fenêtre (71) de la coulisse (position C) et qu'ensuite, elle entraîne le second tube (55) portant le coulisseau (65), l'extrémité arrière du couteau étant basculée.

4. Dispositif selon l'une des revendications 2 ou 3, caractérisé en ce que le mécanisme de commande de la tige de pénétration (48) du tube d'entraînement du couteau (50) et du second tube (55) portant le coulisseau (65) est placé de façon connue en soi dans un boîtier (9) en forme de pistolet, le moteur d'entraînement (2) se trouvant dans la poignée (1) du pistolet.

5. Dispositif selon l'une des revendications 2 à 4, caractérisé en ce que l'arbre du moteur (7, 11) actionne un vilebrequin (16) en vue de la transmission d'un déplacement dans un sens ou dans l'autre à la tige de pénétration (48) sortant à l'avant du boîtier (9), ainsi qu'un engrenage à vis sans fin (27, 29) par l'intermédiaire d'un engrenage démultiplicateur (22, 23), lequel engrenage à vis sans fin (27, 29) actionne le tube d'entraînement du couteau (50) entourant la tige de pénétration (48), par l'intermédiaire d'un accouplement d'arbre débrayable (33) et d'un autre engrenage de démultiplication (35, 53), tandis qu'un interrupteur en forme de détente de pistolet (37), actionnant le moteur (2), provoque simultanément, par l'intermédiaire d'un engrenage à crémaillère (38, 42) démultiplié agissant à l'encontre d'un ressort de rappel (43) au moyen de griffes (57, 58) et d'une bride (56), le déplacement axial du second tube (55) portant le coulisseau (65) pour l'extrémité arrière du couteau.

6. Dispositif selon l'une quelconque des revendications 2 à 5, caractérisé en ce que ses parties faisant saillie hors du boîtier de commande (9) sont démontables, la tige de pénétration (48) apte à être enlevée d'une douille femelle élastique et le tube d'entraînement du couteau (50) apte à être enfoncé au moyen d'un manchon (52, 53), muni d'une denture sur sa périphérie externe, sur le pignon (35) de son arbre d'entraînement (34) sortant par un écrou chapeau (44) démontable, tandis que la griffe (57, 58) entourant la bride (56) du second tube (55) portant le coulisseau (65) est fixée de façon démontable à l'extrémité avant de la tige (42) de l'engrenage à crémaillère (38, 42) sortant à l'extérieur du boîtier (9).

7. Dispositif selon la revendication 1, caractérisé en ce que le couteau (61) est guidé de façon à pouvoir glisser et se tordre, à son extrémité arrière du moyen d'une tête sphérique (63) dans une rainure en forme de queue d'aronde (64) formée sur le côté frontal du coulisseau (65).

8. Dispositif selon la revendication 1, caractérisé en ce que le couteau (61) est entouré, à l'exception de son bord coupant, par une gaine (62) en forme d'enveloppe flexible en matière synthétique.

9. Dispositif selon la revendication 8, caractérisé en ce qu'en vue de compenser la longueur du couteau enveloppé (61) par rapport à l'enveloppe en matière synthétique (62) lorsqu'il se courbe, celui-ci est monté déplaçable longitudinalement à son extrémité arrière, au moyen d'une rainure longitudinale (66), sur un axe transversal (67) de l'enveloppe en matière synthétique (62).

10. Dispositif selon la revendication 8, caractérisé en ce qu'il est prévu, de façon connue en soi, pour permettre l'électrocautérisation, le courant électrique étant amené dans la gaine du couteau (62) dans laquelle est inséré à cet effet un rail conducteur de courant (72) qui est relié au moyen d'une ligne électrique (73) à une source de tension (74).

11. Dispositif selon la revendication 10, caractérisé en ce que le rail conducteur de courant (72) est muni de parties saillantes (75) conductrices, qui s'étendent jusque sur le côté extérieur de la gaine du couteau (62) et sortent uniquement à cet endroit pour faire contact, de sorte qu'une thermocoagulation se produit seulement sur le bord de coupe du tissu non enlevé.

12. Dispositif selon l'une des revendications 10 ou 11, caractérisé en ce que l'alimentation en courant vers la gaine du couteau (62) est interrompue pendant le déplacement du couteau (61) pour l'exécution de l'incision rectiligne et radiale (D).

13. Dispositif selon la revendication 10 ou 11, caractérisé en ce que le côté extérieur de la gaine de couteau (62) a une forme crénelée par rapport au bord coupant du couteau (61) et en ce qu'elle est munie de doigts (77) à l'intérieur desquels s'étendent les parties saillantes conductrices (75) du rail conducteur (72) et de la zone du sommet desquels ces parties saillantes sortent pour entrer en contact avec le tissu.

14. Dispositif selon l'une quelconque des revendications 10 à 13, caractérisé en ce que l'alimentation en courant du rail conducteur (72) est prévue dans la gaine de couteau (62) par l'intermédiaire du coulisseau (65) qui est raccordé par des contacts glissants à la source de tension.

15. Dispositif selon l'une quelconque des revendications 3, 12 ou 14, caractérisé en ce que l'un des pôles de l'alimentation en courant est amené par l'intermédiaire d'un rail de contact (80) ouvert disposé dans la coulisse d'entraînement (69) pour la broche d'entraînement (68), de sorte que le courant est ensuite seulement amené lorsque la broche d'entraînement (68) arrive en contact avec le rail de contact pendant son déplacement, c'est-à-dire si l'on considère le pivotement du couteau (61) sur la trajectoire circulaire conique.

16. Dispositif selon la revendication 11, caractérisé en ce que le rail conducteur (72) forme une seule pièce avec ses parties saillantes conductrices (75), et a en section transversale, la forme d'un U et en ce qu'il est constitué en acier à ressorts stabilisant le couteau flexible (61), les extrémités des parties saillantes (75) qui sortent afin de faire contact avec le tissu, étant élargies notamment en forme de T.

17. Dispositif selon l'une quelconque des revendications 10 à 16, caractérisé en ce que l'alimentation en courant du rail conducteur de courant (72) est réalisée de façon unipolaire dans le dispositif, le second pôle étant amené par une cathode placée sur le corps.

Fig. 5

Fig. 1

0 041 963

0 041 963

Fig. 1a

Fig. 2

Fig. 3

Fig. 4

Fig. 6

Fig. 7

Fig. 8

Fig. 9

13

Fig. 1o

Fig. 11

Fig.13

61 75 77 62a 77 77 78 75 77 78

XIV

XIV

62 72

61 Fig.14

76

75 62a

62b 72

62

Fig.12

78 77

81 77 78

61 61

62 a

48

50

60

0 041 963

17